(19) Europäisches Patentamt
European Patent Office
Office européen des brevets

(11) Veröffentlichungsnummer: **0 459 495 B1**

# (12) EUROPÄISCHE PATENTSCHRIFT

(45) Veröffentlichungstag der Patentschrift: **25.01.95**

(51) Int. Cl.⁶: **C07C 45/46**, C07C 49/84

(21) Anmeldenummer: **91108888.8**

(22) Anmeldetag: **31.05.91**

## (54) Verfahren zur Acylierung von Naphthylethern mit Hilfe von Zeolith-Katalysatoren.

(30) Priorität: **01.06.90 DE 4017681**

(43) Veröffentlichungstag der Anmeldung:
**04.12.91 Patentblatt 91/49**

(45) Bekanntmachung des Hinweises auf die Patenterteilung:
**25.01.95 Patentblatt 95/04**

(84) Benannte Vertragsstaaten:
**BE CH DE FR GB IT LI NL**

(56) Entgegenhaltungen:
**EP-A- 0 282 134**
**EP-A- 0 334 096**
**US-A- 2 475 567**

(73) Patentinhaber: **HOECHST AKTIENGESELLSCHAFT**
**D-65926 Frankfurt (DE)**

(72) Erfinder: **Neuber, Marita, Dr.**
**Schwanheimer Strasse 104**
**W-6000 Frankfurt am Main 71 (DE)**
Erfinder: **Leupold, Ernst Ingo, Dr.**
**Auf der Erlenwiese 61**
**W-6392 Neu-Ansbach (DE)**

EP 0 459 495 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Acylierung von Naphthylethern mit Hilfe von Zeolith-Katalysatoren. Die Produkte, insbesondere 2-Acetyl-6-methoxy-naphthalin, sind wichtige Zwischenprodukte für die Herstellung von Pharmazeutika oder von Monomeren für Polyester.

Es ist bekannt, Naphthylether, wie Methoxynaphthalin, mit Hilfe von Lewis-Säuren, wie $AlCl_3$, als Katalysatoren zu acylieren. Dabei wird ein 1-Naphthylether in der 4-Position acyliert; bei 2-Naphthylethern hängt die Position der Acylierung stark von der Wahl des Lösungsmittels ab: Erfolgt die Reaktion z.B. in Schwefelkohlenstoff, so ist das 1-Acyl-2-alkoxynaphthalin das Hauptprodukt. Wird dagegen beispielsweise in Nitrobenzol gearbeitet, entsteht hauptsächlich 6-Acyl-2-alkoxynaphthalin (Houben-Weyl, Methoden der Organischen Chemie, 4. Auflage, Band VII/2a, S. 71/73 (1973)).

2-Naphthylether lassen sich in wasserfreier Flußsäure mit hoher Selektivität zu den in 6-Position acylierten Produkten umsetzen (US-PS 4593125).

Diese Verfahren haben allerdings eine Reihe von Nachteilen. So müssen die Lewis-Säure-Katalysatoren in mindestens stöchiometrischen Mengen eingesetzt werden, damit die Acylierungsreaktion ablaufen kann. Bei der Aufarbeitung der Reaktionsprodukte wird der Katalysator zerstört, wobei anorganische Salze entstehen. In allen Fällen muß mit Lösungsmitteln gearbeitet werden. Bei der Acylierung in wasserfreier Flußsäure ist der Katalysator gleichzeitig das Lösungsmittel. Nach Abtrennung aus dem Reaktionsprodukt kann die Flußsäure wiederverwendet werden. Die acylierten Produkte müssen neutralisiert werden, wobei ebenfalls Salze anfallen. Darüber hinaus ist wasserfreie Flußsäure äußerst toxisch und korrosiv. Technisch aufwendige Apparaturen aus speziellen Werkstoffen sind erforderlich, um mit Flußsäure arbeiten zu können.

Es bestand daher ein Bedarf für ein verbessertes Verfahren zur Acylierung von Naphthylethern, das sich insbesondere dadurch auszeichnet, daß es einfach zu handhaben ist, in nicht korrosiven und/oder toxischen Medien sowie ohne Lösungsmittel durchzuführen ist. Darüber hinaus soll der Katalysator einfach abzutrennen und wiederverwendbar sein.

Es wurde nun gefunden, daß Naphthylether der Formel I (s. Patentanspruch 1) mit Acylierungsmitteln der Formel $R^2$-CO-X zu acylierten Naphthylethern der Formel II (s. Patentanspruch 1) umgesetzt werden können, wobei $R^1$ und $R^2$ unabhängig voneinander $C_1$- bis $C_{10}$-Alkyl, $C_2$- bis $C_{10}$-Alkenyl oder $C_3$- bis $C_8$-Cycloalkyl und $R^2$ zusätzlich $C_6$- bis $C_{10}$-Aryl bedeuten und X für Cl, Br, $OCOR^2$, OH oder $C_1$- bis $C_3$-Alkoxy steht, indem man als Katalysatoren Zeolithe einsetzt, denen in der wasserfreien und templatfreien Form die allgemeine Formel $Z \cdot Al_2O_3 \cdot x\ SiO_2$ (III) zukommt, in welcher Z $M^I_2O$, $M^{II}O$ und/oder $(M^{III})_2O_3$ bedeutet, worin $M^I$ für ein Alkalimetall, Ammonium oder Wasserstoff, $M^{II}$ für ein Erdalkalimetall und $M^{III}$ für ein seltenes Erdmetall der Ordnungszahl 57 bis 71 im Periodischen System der Elemente, bevorzugt Cer oder Lanthan, steht, und x eine ganze Zahl von 4 bis 4000 bedeutet, mit der Maßgabe, daß mindestens 50 % der negativen Gitterladungen durch Protonen, Ammonium und/oder die anderen unter $M^{II}$ und $M^{III}$ genannten Metallionen kompensiert sind, und deren Poren von mindestens 10 Tetraederatomen gebildet werden.

Es ist zwar bekannt, daß Phenylether, wie Anisol, mit Hilfe von Zeolithen als Katalysatoren acyliert werden können, wobei Umsätze bis zu 75 % und Selektivitäten zu 4-Alkoxyphenylketonen von 98 bis 100 % erzielt werden können (DE-OS 3809260). Dennoch war es nach dem Stand der Technik nicht zu erwarten, daß auch die im Vergleich zu den Phenylethern sehr viel voluminöseren Naphthylether mit Zeolithen als Katalysatoren acyliert werden können. Nach Weisz (Pure Appl. Chem. 52, 2091-2103 (1980)) kann sich im Bereich der Konfigurationsdiffusion (d.h. diffundierende Moleküle und Porenweiten haben vergleichbare Abmessungen) der Diffusions-Koeffizient zweier Moleküle um mehrere Größenordnungen unterscheiden, wenn die Abmessungen der Moleküle nur geringfügig anders sind (z.B. unterscheidet sich der effektive Diffusionskoeffizient von o- und p-Xylol in dem Zeolith ZSM-5 um den Faktor $10^4$, Weisz, a. a. 0.). Der Diffusionskoeffizient hängt von der Temperatur ab. Diese Abhängigkeit kann durch eine der Arrhenius'schen analogen Gleichung beschrieben werden. Die Aktivierungsenergie für die Diffusion wird dabei umso größer, je besser die Abmessungen von Molekülen und Porenweiten übereinstimmen (Weisz, a. a. 0.). Nach diesem Wissensstand war zu erwarten, daß die voluminösen Napthylether sowie die noch sperrigeren acylierten Reaktionsprodukte, wenn überhaupt, so erst bei sehr hohen Temperaturen in den Zeolithporen ausreichend schnell diffundieren können, um befriedigende Umsätze und Ausbeuten zu erhalten.

Überraschenderweise können Naphthylether aber schon bei niedrigen Reaktionstemperaturen umgesetzt werden. So ergab z.B. die Reaktion von 2-Methoxynaphthalin mit Acetanhydrid bei 120 °C in der flüssigen Phase eine Ausbeute von 20,0 % an Acetylmethoxynaphthalinen bei einer Selektivität von 98 %.

Außerdem wurde gefunden, daß bei Einsatz von 2-Naphthylethern die Anteile der beiden hauptsächlich gebildeten Produkte, nämlich die in 1- bzw. 6-Stellung acylierten 2-Naphthylether, durch Variation der

Versuchsbedingungen und des Acylierungsmittels verändert werden können. So erfolgt die Acylierung bevorzugt in 1-Position, wenn mit Säurechloriden umgesetzt wird, und in 6-Position, wenn Carbonsäuren oder Carbonsäure/Carbonsäureanhydrid--Mischungen eingesetzt werden. Die Acylierungsreaktion kann sowohl in der Gasphase als auch in der Flüssigphase durchgeführt werden. Die Flüssigphasenreaktion ist dabei bevorzugt. Beim Arbeiten in der Flüssigphase wird der Anteil des in 6-Stellung acylierten Naphthylethers erhöht, wenn das Acylierungsmittel langsam zugegeben wird und nicht von vornherein im Reaktionsgemisch enthalten ist. In den Beispielen sind Ergebnisse nach diesen unterschiedlichen Verfahrensvarianten enthalten.

Beim Arbeiten in der Gasphase führt eine erhöhte Reaktionstemperatur ebenfalls zur verstärkten Bildung des 6-Acyl-2-alkoxynaphthalins. Auch hierfür sind einige Ergebnisse in den Beispielen angegeben.

Die Reaktion verläuft in der Flüssigphase sehr selektiv. In das Gasphase findet dagegen in gewissem Maße Isomerisierung von Alkoxynapthalinen zu Alkylnaphtholen, Disproportionierung von Alkoxynaphthalinen, hydrolytische Spaltung der Reaktanden sowie die Bildung von Naphthylestern statt. Bei höheren Temperaturen können bei den Gasphasenreaktionen auch Umsetzungen der Carbonsäurederivate ablaufen.

Geeignete Reste $R^1$ und $R^2$ sind z.B. Methyl, Äthyl, Propyl, Isopropyl, Butyl, Isobutyl, die isomeren Amyle, Hexyle, Octyle, Nonyle, Decyle, sowie Vinyl, Propenyl, Butenyl, die isomeren Amylene, Hexene, Octene und Decene.

Spezielle Naphthylether (I) für das erfindungsgemäße Verfahren sind beispielsweise 1- und 2-Methoxynaphthalin, 1- und 2-Äthoxynaphthalin, 1- und 2-Propoxynaphthalin, Cyclohexyl-1-naphthylether, Cyclohexyl-2-naphthylether.

Acylierungsmittel für das erfindungsgemäße Verfahren sind beispielsweise Essigsäure, Essigsäureanhydrid, Essigsäurechlorid, Essigsäurebromid, Essigsäuremethylester, Propionsäure, Propionsäureanhydrid, Propionsäurechlorid, Buttersäure, Buttersäureanhydrid, Buttersäurechlorid, Isobuttersäure, Isobuttersäureanhydrid, Isobuttersäurechlorid, Pivalinsäurechlorid, Pivalinsäureanhydrid, Valeriansäure, Valeriansäurechlorid, Valeriansäureanhydrid. Dabei sind Carbonsäureanhydride oder Mischungen aus Carbonsäuren und ihren Anhydriden bevorzugt.

Als Katalysatoren für das erfindungsgemäße Verfahren eignen sich Zeolithe wie in Formel (III) definiert, deren Porenöffnungen aus mindestens 10 Tetraederatomen gebildet werden. Als Tetraederatome werden Si und Al bezeichnet, die tetraedrisch von Sauerstoffatomen umgeben sind. Diese Tetraeder sind über gemeinsame Sauerstoffatome verknüpft und bilden eine Kristallstruktur, die von definierten Poren und Hohlräumen durchzogen ist. Die Porenweiten und -formen hängen vom Zeolithtyp ab (siehe z.B. Atlas of Zeolite Structure Types, W.M. Meier und D.H. Olson, 1987).

Es sind jedoch auch Zeolithe geeignet, bei denen ein Teil des Aluminiums bzw. Siliziums durch andere Gitteratome, vorzugsweise Bor, Eisen, Gallium, Germanium, Titan und/oder Zirkon ersetzt ist.

Die negativen Ladungen der $AlO_4^-$ -Tetraeder sind durch austauschbare Kationen wie z.B. $H^+$, $Na^+$, $K^+$, $Ca^{2+}$, $Mg^{2+}$ oder organische Kationen wie $N^+R_4$ und $P^+R_4$ kompensiert.

Für das erfindungsgemäße Verfahren geeignete Zeolithe sind z.B. die Zeolithe ZSM-5 (US-PS 3 702 886), ZSM-11 (US-PS 3 709 979), ZSM-12 (US-PS 3 970 544), ZSM-20 (US-PS 3 972 983), Beta (US-PS 3 308 069), EU-1 (EP 042 226), Y, L, Offretit oder Mordenit (beschrieben in D.W. Breck, "Zeolite Molecular Sieves", 1974).

Außerdem können Molekularsiebe auf der Basis von Aluminiumphosphat, bei denen ein Teil der Tetraederatome z.B. durch Si, Co, Mg und/oder Mn ersetzt sind, verwendet werden.

Die genannten Zeolithe können nach Vorschriften aus der Literatur durch hydrothermale Synthese hergestellt werden. Nach der Kristallisation werden die Zeolithe abfiltriert, getrocknet und in oxidierender Atmosphäre, bevorzugt an Luft, calciniert, um das organische Templat aus den Poren zu entfernen. (Die templatfreie Form ist frei von Alkylammonium- oder -phosphoniumionen sowie von Aminen). Eventuell vorhandene Alkalimetall-Ionen (in diesem Fall steht Z in Formel (III) für $M_2O$) werden anschließend durch Ionenaustausch gegen zwei- oder dreiwertige Ionen der Erdalkalimetalle oder Seltenen Erdmetalle oder gegen Ammonium-Ionen oder Protonen ausgetauscht. Dabei ist der Ionenaustausch mit $NH_4^+$ oder $H^+$ ganz besonders bevorzugt. Diese saure Modifizierung ist notwendig, weil der Zeolith sonst keine katalytische Wirkung entfaltet. Dabei ist es zweckmäßig, daß mindestens 50 %, vorzugsweise mindestens 75 % der Alkalimetallionen durch die genannten anderen Ionen ausgetauscht sind. Bei 200 bis 800 °C, bevorzugt bei 400 bis 550 °C werden die Zeolithe durch Dehydratisierung (und Deammonisierung bei $NH_4^+$-Formen) in die katalytisch aktive Form überführt. Das $SiO_2/Al_2O_3$-Verhältnis der Zeolithe kann in einem weiten Bereich, z.B. zwischen 4 und 4000 - je nach Zeolith-Typ - variieren. Der Aluminiumgehalt der erfindungsgemäß verwendeten Zeolithe kann zum Zwecke einer weitergehenden Modifizierung durch Behandlung mit Mineralsäuren, organischen Säuren oder chelatisierenden Substanzen innerhalb der genannten Grenzen vermindert werden. Bevorzugt sind $SiO_2/Al_2O_3$-Verhältnisse zwischen 20 und 300. Die Kristallitgröße kann z.B.

zwischen 0,01 und 10 µm, bevorzugt zwischen 0,05 und 0,1 µm, liegen.

Die Zeolithe werden für die erfindungsgemäße Verwendung vorteilhaft mit Hilfe von Bindern in eine geeignete Anwendungsform, z.B. in Strangform gebracht. Als Binder sind vor allem Oxide, Hydroxyde oder Hydroxychloride des Aluminiums und die Oxide des Siliziums, Titans und Zirkons sowie Tonmaterialien geeignet.

Das Acylierungsmittel kann z.B. in einem Verhältnis von 0,1 bis 20 mol, bevorzugt von 0,5 bis 5 mol pro mol Naphthylether eingesetzt werden. Die Reaktionstemperaturen können zwischen 30 und 500 ° C, bevorzugt zwischen 100 und 300 ° C, liegen. Dabei ist es günstig, für die Flüssigphasenacylierung Temperaturen im unteren Bereich zwischen 30 und 200 ° C, bevorzugt zwischen 120 und 170 ° C, zu wählen und für die Gasphasenacylierung Temperaturen im oberen Temperaturbereich zwischen 150 und 500 ° C, bevorzugt zwischen 200 und 300 ° C, anzuwenden.

Der Druck ist für den Reaktionsverlauf wenig entscheidend. Er kann auf Werte zwischen 0,5 und 100 bar, bevorzugt zwischen 1 und 10 bar, eingestellt werden. Am günstigsten arbeitet man bei Atmosphärendruck. Für die Reaktion in der Flüssigphase kann es zur Erreichung einer Reaktionstemperatur, die höher als die Siedetemperatur des Reaktionsgemisches ist, notwendig sein, unter Druck zu arbeiten.

Die Acylierung in der Flüssigphase kann in allen geeigneten Apparaturen durchgeführt werden, am einfachsten in einem Rührkessel mit pulverförmigem, suspendiertem Katalysator.

Die Reaktanden können dabei zusammen mit dem Katalysator auf die Reaktionstemperatur gebracht werden. In bestimmten Fällen ist es günstiger, nach Erreichen der Reaktionstemperatur einen der Reaktionspartner, bevorzugt das Acylierungsmittel, langsam zuzugeben. Die Reaktion kann in Abwesenheit oder in Gegenwart von gegenüber den Reaktionsteilnehmern und Katalysatoren inerten Lösungsmitteln wie Nitrobenzol, durchgeführt werden.

Bezogen auf die Masse an eingesetzten Reaktanden ist es günstig, zwischen 0,5 und 100 Gew.-% Katalysator, bevorzugt zwischen etwa 1 und 10 Gew.-%, einzusetzen. Die Reaktionsdauer kann zwischen 0,5 h und mehreren Tagen, bevorzugt zwischen 2 und 10 Stunden, betragen. Nach erfolgter Reaktion in der flüssigen Phase kann der Zeolith auf einfache Weise durch Filtration aus dem Reaktionsgemisch abgetrennt werden.

Für die Durchführung der Reaktion in der Gasphase können prinzipiell alle für Gasphasenreaktionen geeigneten Apparaturen verwendet werden. Technisch am einfachsten ist ein Festbett-Strömungsreaktor zu handhaben. Der Katalysator kann dabei in Form von Pellets in den Reaktor eingebaut werden. Für die Herstellung der Pellets kann der Zeolith zusammen mit einem Bindermaterial wie $Al_2O_3$ oder $SiO_2$ oder aber binderfrei verpreßt werden.

Die Reaktanden können flüssig in den Reaktor eindosiert werden, wobei ein bei Raumtemperatur fester Naphthylether aufgeschmolzen oder in einem inerten Lösungsmittel oder in einem Überschuß an Acylierungsmittel gelöst werden kann. Wenn man in der Gasphase arbeiten will, werden die Reaktanden vor dem Katalysatorbett verdampft. Sie können auch vor dem Reaktor durch geeignete Maßnahmen in die Gasphase überführt und dann über den Katalysator geleitet werden. Dabei können die Reaktanden allein oder im Gemisch mit einem hinsichtlich der Reaktion inerten Gas, wie Stickstoff oder Wasserstoff, eingesetzt werden. Die Produkte werden nach dem Reaktor kondensiert.

Die Verweilzeit der Reaktanden kann zwischen 0,05 und 20 s, bevorzugt zwischen 1 und 10 s, liegen. Die Raumgeschwindigkeiten (LHSV = liquid hourly space velocity) können im Bereich von 0,1 bis 5 $h^{-1}$ eingestellt werden, wobei der Bereich von 0,5 bis 2 $h^{-1}$ besonders günstig ist.

Die Katalysatoren sind mehrfach für die Reaktion einsetzbar. Sie können durch einfaches Calcinieren in oxidierender Atmosphäre, insbesondere in Luft oder Luft/Stickstoffgemischen, bei Temperaturen zwischen 350 und 800 ° C, bevorzugt zwischen 500 und 600 ° C, wieder regeneriert werden.

Das bei der Reaktion erhaltene Gemisch kann auf herkömmliche Art und Weise getrennt werden. Nicht umgesetzte Reaktanden können abdestilliert und wieder für die Acylierungsreaktion verwendet werden. Die entstehenden isomeren acylierten Produkte lassen sich in der Regel durch eine Destillation grob trennen; durch weitere Reinigungsschritte wie Destillation und Umkristallisieren lassen sie sich bis zum gewünschten Grad reinigen.

Die nachstehenden Beispiele sollen das erfindungsgemäße Verfahren näher erläutern.

**Beispiele**

Die katalytischen Versuche in der Flüssigphase wurden in einem Rührkessel durchgeführt. Der Zeolith wurde pulverförmig eingesetzt und vor der Reaktion im Vakuum bei 250 ° C 1 h lang getrocknet. Die Reaktanden wurden außer in den Beispielen 13 und 14 in äquimolarem Verhältnis eingesetzt. In Tabelle 1 sind einige Ergebnisse der Flüssigphasenacylierung von 2-Methoxynaphthalin (2-MO-Np) zusammengestellt.

Die Gasphasenversuche wurden in einem Festbett-Strömungsreaktor bei Atmosphärendruck durchgeführt. 2-Methoxynaphthalin wurde in einem Überschuß an Acylierungsmittel gelöst, das Reaktandengemisch wurde flüssig zudosiert und vor dem Katalysatorbett verdampft. Als Trägergas wurde Stickstoff verwendet. Die Produkte wurden nach dem Reaktor nach verschiedenen Laufzeiten des Katalysators kondensiert und gaschromatographisch analysiert. Der Katalysator wurde in Form von Strängen eingesetzt, die 21 Gew.-% $SiO_2$ als Binder enthielten. Einige Ergebnisse sind in Tabelle 2 zusammengestellt.

Tabelle 1: Acylierung von 2-Methoxynaphthalin, Flüssigphase

| Beispiel | Zeolith | Acylierungsmittel [c] | Temp. °C | Dauer h | Produktausbeuten [d], mol % Acyl-2-MO-Np | Sonst. | Isomerenverhältnis 1- | 6- Acyl-2-MO-Np | Sonst. |
|---|---|---|---|---|---|---|---|---|---|
| 1 | - | $Ac_2O$ | 159 | 4,0 | 0 | 0 | | | |
| 2 | HEU-1 | AcCl | 51-70 | 13,5 | 26 | 0 | 97 | 2 | 1 |
| 3 | HEU-1 | $Ac_2O$ | 155 | 6,5 | 14 | 0 | 80 | 16 | 4 |
| 4 | HBeta | $Ac_2O$ | 154 | 2,5 | 22 | 0 | 70 | 18 | 12 |
| 5 | HBeta | $Ac_2O$/AcOH | 139 | 4,5 | 16 | 1 | 55 | 39 | 6 |
| 6 | HBeta | $Ac_2O$, zutropfen | 154 | 2,0 | 16 | 1 | 60 | 34 | 6 |
| | | | | 10,5 | 18 | 1 | 65 | 29 | 6 |
| 7 | HZSM-12[a] | $Ac_2O$, zutropfen | 158 | 6,0 | 25 | 0 | 83 | 15 | 2 |
| 8 | HZSM-12[b] | $Ac_2O$, zutropfen | 158 | 6,0 | 9 | 0 | 80 | 20 | 0 |
| 9 | HBeta | Propionylchlorid | 133-122 | 3,7 | 35 | 3 | 87 | 13 | 0 |
| 10 | HBeta | Propionsäureanhydrid | 175 | 6,0 | 18 | 2 | 73 | 26 | 1 |
| 11 | HZSM-12[a] | Propionsäureanhydrid | 175 | 6,0 | 19 | 1 | 74 | 25 | 1 |
| 12 | HZSM-12[b] | Propionsäureanhydrid | 175 | 2,0 | 11 | 1 | 67 | 31 | 2 |
| 13 | HBeta | $Ac_2O$[e], zutropfen | 155 | 2,5 | 12 | 2 | 56 | 44 | 0 |
| 14 | HBeta | $Ac_2O$[f], zutropfen | 155 | 5,5 | 27 | 1 | 68 | 32 | 0 |

a) Kristallitgröße 0,05 µm b) Kristallitgröße 0,5 µm c) Ac = $CH_3CO$

d) nur bezogen auf Produkte aus 2-MO-Np e) Mengenverhältnis 2-MO-Np: $Ac_2O$ = 1:0,5

f) Mengenverhältnis 2-MO-Np:$Ac_2O$ = 1:3

**Tabelle 2**: Acylierung von 2-Methoxynaphthalin, Gasphase

| Beispiel | Zeolith | Acylierungsmittel a) | mol pro mol 2-MO-Np | Temp. °C | LHSV $h^{-1}$ | Laufzeit h | Produktausbeuten b), mol % Acyl-2-MO-Np | Sonst. | Isomerenverhältnis 1-Acyl-2-MO-Np | 6-Acyl-2-MO-Np | Sonst. |
|---|---|---|---|---|---|---|---|---|---|---|---|
| 15 | HEU-1 | AcOH | 8 | 250 | 1 | 0,5-1 | 1 | 1 | 10 | 90 | 0 |
| 16 | HEU-1 | AcCl | 10 | 250 | 1 | 0,5-1 | 6 | 3 | 77 | 23 | 0 |
| 17 | HEU-1 | $Ac_2O$ | 5 | 250 | 1 | 0,5-1 | 4 | 4 | 66 | 34 | 0 |
| 18 | HZSM-12 | Propionsäureanhydrid | 4 | 250 | 1 | 0 - 0,5 | 11 | 2 | 19 | 80 | 1 |
| | | | | | | 2,5-4,5 | 9 | 2 | 16 | 83 | 1 |
| 19 | HZSM-12 | Propionsäureanhydrid | 4 | 200 | 1 | 0,5-1 | 11 | 2 | 54 | 44 | 2 |
| 20 | HZSM-12 | Propionsäureanhydrid | 4 | 300 | 1 | 0,5-1 | 15 | 2 | 9 | 89 | 2 |
| 21 | HZSM-12 | Propionsäure | 8 | 300 | 1 | 0,5-1 | 10 | 5 | 2 | 94 | 4 |

a) Ac = $CH_3CO$ b) nur bezogen auf Produkte aus 2-MO-Np

## Patentansprüche

1. Verfahren zur Acylierung von Naphthylethern der Formel

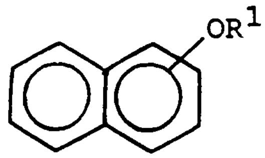

(I)

mit Acylierungsmitteln der Formel $R^2$-CO-X zu acylierten Naphthylethern der Formel

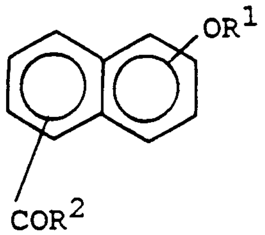

(II)

wobei $R^1$ und $R^2$ unabhängig voneinander $C_1$- bis $C_{10}$-Alkyl, $C_2$- bis $C_{10}$-Alkenyl oder $C_3$- bis $C_8$-Cycloalkyl und $R^2$ zusätzlich $C_6$- bis $C_{10}$-Aryl bedeuten und X für Cl, Br, -OCOR$^2$, OH oder $C_1$- bis $C_3$-Alkoxy steht, dadurch gekennzeichnet, daß die Umsetzung in Gegenwart von Zeolith-Katalysatoren durchgeführt wird, denen in der wasserfreien und templatfreien Form die allgemeine Formel $Z \cdot Al_2O_3 \cdot x\ SiO_2$ (III) zukommt, in welcher Z $M^I_2O$, $M^{II}O$ und/oder $(M^{III})_2O_3$ bedeutet, worin $M^I$ für ein Alkalimetallatom, Ammonium oder ein Wasserstoffatom, $M^{II}$ für ein Erdalkalimetallatom und $M^{III}$ für ein seltenes Erdmetall der Ordnungszahl 57 bis 71 im Periodischen System der Elemente steht, und x eine Zahl von 4 bis 4000 bedeutet, mit der Maßgabe, daß mindestens 50 % der negativen Gitterladungen durch Protonen, Ammonium und/oder die anderen unter $M^{II}$ und $M^{III}$ genannten Metallionen kompensiert sind, und deren Poren aus mindestens 10 Tetraederatomen gebildet werden.

2. Verfahren nach Anspruch 1, dadurch gekennzeichnet, daß falls Z für $M_2O$ steht, mindestens 75 % der Alkalimetallatome durch Wasserstoffatome, Ammonium, Erdalkalimetallatome und/oder Seltene Erdmetallatome ausgetauscht sind.

3. Verfahren nach Anspruch 1 oder 2, dadurch gekennzeichnet, daß x in der Formel (III) eine Zahl von 20 bis 300 bedeutet.

4. Verfahren nach einem oder mehreren der Ansprüche 1 bis 3, dadurch gekennzeichnet, daß die Kristallitgröße der Zeolithe zwischen 0,01 und 10 $\mu$m, bevorzugt zwischen 0,05 und 0,1 $\mu$m, liegt.

5. Verfahren nach einem oder mehreren der Ansprüche 1 bis 4, dadurch gekennzeichnet, daß die Reaktion bei Temperaturen von 30 bis 500 °C, bevorzugt von 100 bis 300 °C, durchgeführt wird.

6. Verfahren nach einem oder mehreren der Ansprüche 1 bis 5, dadurch gekennzeichnet, daß die Reaktion in der flüssigen Phase durchgeführt wird.

7. Verfahren nach Anspruch 6, dadurch gekennzeichnet, daß die Reaktion bei Temperaturen von 30 bis 200 °C, bevorzugt von 120 bis 170 °C, durchgeführt wird.

8. Verfahren nach einem oder mehreren der Ansprüche 1 bis 4, dadurch gekennzeichnet, daß die Reaktion in der Gasphase stattfindet.

**9.** Verfahren nach Anspruch 8, dadurch gekennzeichnet, daß die Reaktion bei Temperaturen von 150 bis 500 °C, bevorzugt von 200 bis 300 °C, durchgeführt wird.

**10.** Verfahren nach einem oder mehreren der Ansprüche 1 bis 9, dadurch gekennzeichnet, daß die Reaktion im Druckbereich von 0,5 bis 100 bar, bevorzugt von 1 bis 10 bar, durchgeführt wird.

**11.** Verfahren nach einem oder mehreren der Ansprüche 1 bis 10, dadurch gekennzeichnet, daß 0,1 bis 20 mol Acylierungsmittel pro mol Naphthylether, bevorzugt 0,5 bis 5 mol, eingesetzt werden.

**12.** Verfahren nach einem oder mehreren der Ansprüche 1 bis 11, dadurch gekennzeichnet, daß als Acylierungsmittel Carbonsäureanhydride oder Mischungen aus Carbonsäuren und ihren Anhydriden eingesetzt werden.

**13.** Verfahren nach einem oder mehreren der Ansprüche 1 bis 12, dadurch gekennzeichnet, daß 2-Naphthylether, insbesondere 2-Methoxynaphthalin, acyliert werden.

**14.** Verfahren nach einem oder mehreren der Ansprüche 1 bis 13, dadurch gekennzeichnet, daß bezogen auf die Masse an eingesetzten Reaktanden zwischen 0,5 und 100 Gew.-% Katalysator, bevorzugt zwischen 1 und 10 Gew.-% Katalysator, eingesetzt werden.

**15.** Verfahren nach einem oder mehreren der Ansprüche 1 bis 7 und 10 bis 14, dadurch gekennzeichnet, daß das Acylierungsmittel bei Reaktionstemperatur langsam zur Naphthylether/Katalysator-Suspension gegeben wird.

## Claims

**1.** A process for the acylation of a naphthyl ether of the formula

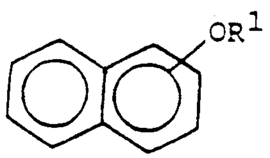

(I)

using an acylating agent of the formula $R^2$-CO-X to give an acylated naphthyl ether of the formula

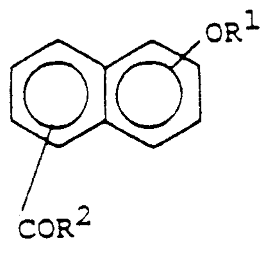

(II)

in which $R^1$ and $R^2$ independently of one another are $C_1$- to $C_{10}$-alkyl, $C_2$- to $C_{10}$-alkenyl or $C_3$- to $C_8$-cycloalkyl and $R^2$ is additionally $C_6$- to $C_{10}$-aryl and X is Cl, Br, - $OCOR^2$, OH or $C_1$- to $C_3$-alkoxy, which comprises carrying out the reaction in the presence of zeolite catalysts which have the formula $Z \cdot Al_2O_3 \cdot x\ SiO_2$ (III) in the anhydrous and template-free form, in which Z is $M^I{}_2O$, $M^{II}O$ and/or $(M^{III})_2O_3$, in which $M^I$ is an alkali metal atom, ammonium or a hydrogen atom, $M^{II}$ is an alkaline earth metal atom and $M^{III}$ is a rare earth metal of atomic number 57 to 71 in the periodic table of the elements, and x is a number from 4 to 4000, with the proviso that at least 50 % of the negative lattice charges are compensated by protons, ammonium and/or the other metal ions mentioned under $M^{II}$ and $M^{III}$, and the pores of which are formed from at least 10 tetrahedral atoms.

2. The process as claimed in claim 1, wherein if Z is $M_2O$, at least 75 % of the alkali metal atoms are replaced by hydrogen atoms, ammonium, alkaline earth metal atoms and/or rare earth metal atoms.

3. The process as claimed in claim 1 or 2, wherein x in the formula (III) is a number from 20 to 300.

4. The process as claimed in one or more of claims 1 to 3, wherein the crystallite size of the zeolites is between 0.01 and 10 µm, preferably between 0.05 and 0.1 µm.

5. The process as claimed in one or more of claims 1 to 4, wherein the reaction is carried out at temperatures of 30 to 500 °C, preferably of 100 to 300 °C.

6. The process as claimed in one or more of claims 1 to 5, wherein the reaction is carried out in the liquid phase.

7. The process as claimed in claim 6, wherein the reaction is carried out at temperatures of 30 to 200 °C, preferably of 120 to 170 °C.

8. The process as claimed in one or more of claims 1 to 4, wherein the reaction takes place in the gas phase.

9. The process as claimed in claim 8, wherein the reaction is carried out at temperatures of 150 to 500 °C, preferably of 200 to 300 °C.

10. The process as claimed in one or more of claims 1 to 9, wherein the reaction is carried out in the pressure range from 0.5 to 100 bar, preferably from 1 to 10 bar.

11. The process as claimed in one or more of claims 1 to 10, wherein 0.1 to 20 mol of acylating agent are employed per mol of naphthyl ether, preferably 0.5 to 5 mol.

12. The process as claimed in one or more of claims 1 to 11, wherein carboxylic anhydrides or mixtures of carboxylic acids and their anhydrides are employed as acylating agents.

13. The process as claimed in one or more of claims 1 to 12, wherein 2-naphthyl ethers, in particular 2-methoxynaphthalene, are acylated.

14. The process as claimed in one or more of claims 1 to 13, wherein between 0.5 and 100 % by weight of catalyst, preferably between 1 and 10 % by weight of catalyst, are employed relative to the weight of reactants employed.

15. The process as claimed in one or more of claims 1 to 7 and 10 to 14, wherein the acylating agent is added slowly at reaction temperature to the naphthyl ether/catalyst suspension.

**Revendications**

1. Procédé pour acyler des oxydes de naphtyle de formule

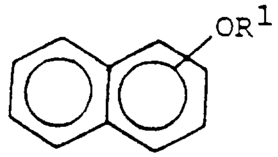

(I)

avec des agents d'acylation de formule $R^2$-CO-X, pour obtenir des oxydes de naphtyle de formule

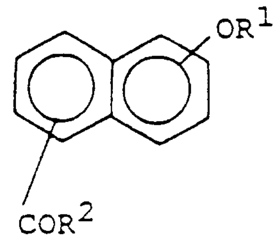

(II)

où $R^1$ et $R^2$, indépendamment l'un de l'autre, sont chacun un radical alkyle en $C_1$ à $C_{10}$, alcényle en $C_2$ à $C_{10}$ ou cycloalkyle en $C_3$ à $C_8$, et $R^2$ est de plus un radical aryle en $C_6$ à $C_{10}$, et X est Cl, Br, $OCOR^2$, OH ou un radical alcoxy en $C_1$ à $C_3$, caractérisé en ce qu'on met en oeuvre la réaction en présence de catalyseurs zéolitiques, qui ont, sous leur forme anhydre et sans gabarit, la formule générale $Z.Al_2O_3.x\ SiO_2$ (III), où Z est $M_2{}^IO$, $M^{II}O$ et/ou $(M^{III})_2O_3$, où $M^I$ est un métal alcalin, l'ammonium ou l'hydrogène, $M^{II}$ est un métal alcalinoterreux et $M^{III}$ est un métal des terres rares ayant un numéro atomique de 57 à 71 dans le Système Périodique des Eléments, de préférence le cérium ou le lanthane, et x est un nombre entier de 4 à 4000, du moment qu'au moins 50 % des charges réticulaires négatives sont compensées par des protons, de l'ammonium et/ou les autres ions métalliques mentionnés pour $M^{II}$ et $M^{III}$, et dont les pores sont constitués d'au moins 10 atomes de tétraèdre.

**2.** Procédé selon la revendication 1, caractérisé en ce que, quand Z est $M_2O$, au moins 75 % des atomes de métaux alcalins sont remplacés par des atomes d'hydrogène, de l'ammonium, des atomes de métaux alcalinoterreux et/ou des atomes de métaux des terres rares.

**3.** Procédé selon la revendication 1 ou 2, caractérisé en ce que x, dans la formule (III), est un nombre de 20 à 300.

**4.** Procédé selon l'une ou plusieurs des revendications 1 à 3, caractérisé en ce que la taille des cristallites de zéolite est comprise entre 0,01 et 10 $\mu$m et de préférence entre 0,05 et 0,1 $\mu$m.

**5.** Procédé selon l'une ou plusieurs des revendications 1 à 4, caractérisé en ce que la réaction est mise en oeuvre à des températures de 30 à 500 °C et de préférence de 100 à 300 °C.

**6.** Procédé selon l'une ou plusieurs des revendications 1 à 5, caractérisé en ce que la réaction est mise en oeuvre en phase liquide.

**7.** Procédé selon la revendication 6, caractérisé en ce que la réaction est mise en oeuvre à des températures de 30 à 200 °C et de préférence de 120 à 170 °C.

**8.** Procédé selon l'une ou plusieurs des revendications 1 à 4, caractérisé en ce que la réaction a lieu en phase gazeuse.

**9.** Procédé selon la revendication 8, caractérisé en ce que la réaction est mise en oeuvre à des températures de 150 à 500 °C et de préférence de 200 à 300 °C.

**10.** Procédé selon l'une ou plusieurs des revendications 1 à 9, caractérisé en ce que la réaction est mise en oeuvre sur l'intervalle de pressions de 0,5 à 100 bar et de préférence de 1 à 10 bar.

**11.** Procédé selon l'une ou plusieurs des revendications 1 à 10, caractérisé en ce qu'on utilise de 0,1 à 20 mol et de préférence de 0,5 à 5 mol d'agent d'acylation par mode d'oxyde de naphtyle.

**12.** Procédé selon l'une ou plusieurs des revendications 1 à 11, caractérisé en ce qu'on utilise comme agent d'acylation des anhydrides carboxyliques ou des mélanges d'acides carboxyliques et de leurs anhydrides.

**13.** Procédé selon l'une ou plusieurs des revendications 1 à 12, caractérisé en ce qu'on acyle un oxyde de 2-naphtyle, en particulier le 2-méthoxynaphtalène.

**14.** Procédé selon l'une ou plusieurs des revendications 1 à 13, caractérisé en ce que, par rapport à la masse des réactifs utilisés, on utilise de 0,5 à 100 et de préférence de 1 à 10 % en poids de catalyseur.

**15.** Procédé selon l'une ou plusieurs des revendications 1 à 7 et 10 à 14, caractérisé en ce que l'agent d'acylation est lentement ajouté, à la température de la réaction, à la suspension de l'oxyde de naphtyle et du catalyseur.